(19) Europäisches Patentamt
European Patent Office
Office européen des brevets

(11) EP 3 720 563 B1

(12) **EUROPEAN PATENT SPECIFICATION**

(45) Date of publication and mention of the grant of the patent:
**07.01.2026 Bulletin 2026/02**

(21) Application number: **18819495.5**

(22) Date of filing: **29.11.2018**

(51) International Patent Classification (IPC):
***A61Q 11/00*** *(2006.01)* ***A61K 8/81*** *(2006.01)*

(52) Cooperative Patent Classification (CPC):
**A61Q 11/00; A61K 8/8182;** A61K 2800/5424

(86) International application number:
**PCT/US2018/063002**

(87) International publication number:
**WO 2019/112869 (13.06.2019 Gazette 2019/24)**

(54) **ORAL CARE COMPOSITIONS**

MUNDPFLEGEZUSAMMENSETZUNGEN

COMPOSITIONS DE SOINS BUCCODENTAIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.12.2017 US 201715834537**

(43) Date of publication of application:
**14.10.2020 Bulletin 2020/42**

(73) Proprietor: **Kenvue Brands LLC**
**Summit, NJ 07901 (US)**

(72) Inventors:
- **QUEIROZ, Daniel**
  **Skillman**
  **New Jersey 08558 (US)**
- **TESTER, Chantel**
  **Skillman**
  **New Jersey 08558 (US)**
- **GIANO, Michael C.**
  **Skillman**
  **New Jersey 08558 (US)**
- **CHEN, Rebecca**
  **Princeton**
  **New Jersey 08540 (US)**
- **GAMBOGI, Robert J.**
  **Skillman**
  **New Jersey 08558 (US)**

(74) Representative: **Carpmaels & Ransford LLP**
**One Southampton Row**
**London WC1B 5HA (GB)**

(56) References cited:

| | |
|---|---|
| **EP-A1- 0 897 709** | **WO-A1-2005/027862** |
| **WO-A1-2016/173871** | **JP-A- H06 157 270** |
| **JP-B2- 2 992 415** | **US-A1- 2008 305 457** |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 720 563 B1

## Description

## FIELD OF THE INVENTION

The present invention relates to compositions comprising anionic PVP copolymers for use in oral care composition to prevent demineralization. The present invention relates to phosphorous-containing copolymers of vinyl pyrrolidone used to deliver demineralization prevention benefits.

## BACKGROUND OF THE INVENTION

Caries is one of the most prevalent diseases affecting individuals globally. It is caused by the complex relationship between dental biofilms (plaque), the host and fermentation of dietary sugars to organic acids. Essentially, the acids produced effectively decrease the pH of the microenvironment below 5.5 resulting in enamel dissolution or demineralization. Rapid adjustment to near neutral pH occurs due to the high buffer capacity of saliva resulting in remineralization at the surface of enamel. An imbalance in de- and remineralization cycles, with more time spent in the demineralization, result in caries.

Fluoride, renowned for prevention and repair of tooth decay, has been considered as a possible solution to regulated dental erosion. Recently, different types of micro-/nanoparticles (hydroxyapatite, bioglass, silica particles, etc.) have also been explored for their anti-erosion properties. Some divalent cations, such as calcium, zinc and stannous, have also been used to prevent demineralization.

While fluoride has been widely used in consumer products, almost half of the population still suffers from caries. Micro-/nanoparticles show some efficacy in remineralization, but they are very challenging to formulate in mouth rinse and the efficacy highly depends on the retention time on enamel surface. For example, bioglass, such as that trademarked under the name NOVAMIN, (Novamin, etc.) can only be prepared in anhydrous formulations. Divalent cations also have limitations in either formulation or consumer experience, for example, zinc has a bitter after taste and stannous is known for causing teeth staining issue.

Preventing the demineralization of teeth is rapidly gaining attention as a valid strategy to reduce occurrence and/or progression of dental decay. Hence, there is always a need to develop oral care compositions to prevent demineralization.

WO2005027862A1 relates to oral care compositions comprising a polymer obtainable by copolymerising a mixture of comonomers, said mixture comprising: (a) a cationic monomer selected from (ar-vinylbenzyl) trimethylammonium chloride, (dimethylaminopropyl) methacrylamide, [2(methacryloyloxy)ethyl]trimethylammonium chloride, 2-aminoethylmethacrylate hydrochloride and mixtures thereof; and (b) at least one anionic or neutral monomer selected from styrene, mono-2-(methacryloyl)ethyl succinate, vinyl acetate, N,N-dimethylacrylamide, 2-ethylhexylacrylate, vinylphosphonic acid, acrylic acid, 2-acrylamido-2-methyl-1-propanesulfonic acid, N-[tris(hydroxymethyl)methyl] acrylamide, N-vinylpyrrolidone, butyl acrylate, 2-hydroxyethylacrylate, polyethyleneglycol methylethermethacrylate and mixtures thereof, said oral care composition in the form of any one of a toothpaste, gel, foam, chewing gum, deformable strip or mouthwash and which is suitable for use in the oral cavity.

US2008305457A1 relates to a method of applying a dental agent to a dental surface, the method including: applying a retentive polymeric coating to a dental surface; and subsequently contacting the retentive polymeric coating with a loading composition that includes a dental agent, wherein the contacting occurs under conditions effective to incorporate the dental agent into the retentive polymeric coating for release over time, as well as polymers that include a hydrophobic segment, a hydrophilic segment, a quaternary amine segment, and an alkoxy silane crosslinkable segment; and polymers that include a hydrophobic segment, a hydrophilic segment, a silicon-containing macromer segment, and an alkoxy silane crosslinkable segment.

WO2016173871A1 relates to the use of a composition which contains a peroxide compound and a specific, generically defined copolymer, for bleaching teeth.

JP2992415B2 and JPH06157270A relate to a cosmetic for beautifying skin and hair wherein the cosmetic is obtained by blending a copolymer of 2-methacryloyloxyethylphosphorylcholine and a hydrophilic monomer (preferably having >=5,000 molecular weight) in an amount of preferably 0.001-10wt.% based on the total amount of the cosmetic with other ingredients and is capable of improving the skin and hair conditions such as skin roughening and insufficient gloss and enhancing the moisture retention without any unfavorable effects to touch.

EP0897709A1 relates to a dental coating composition comprising a polymer which has in its molecule one or more phosphorus series acid residues selected from the group consisting of phosphoric acid, phosphonic acid and phosphinic acid and halides and salts thereof and has a weight-average molecular weight of 10,000 to 5,000,000, and water or a lower alcohol, wherein the composition is a dental coating agent which does not easily peel off when drinking or eating, can be removed easily if necessary and does not have a safety problem.

## SUMMARY OF THE INVENTION

The invention relates to compositions comprising an orally-acceptable carrier and an anionic copolymer derived from the polymerization of N-vinyl pyrrolidone (VP) with an anionic monomer compound containing phosphorus as defined in claim 1. Another aspect relates to uses of such compounds in the oral cavity to inhibit demineralization of a tooth.

## DETAILED DESCRIPTION OF THE INVENTION

The compositions of the present invention can comprise, consist of, or consist essentially of the essential elements and limitations of the invention described herein, as well any of the additional or optional ingredients, components, or limitations described herein. The term "comprising" (and its grammatical variations) as used herein is used in the inclusive sense of "having" or "including" and not in the exclusive sense of "consisting only of."

The terms "a" and "the" as used herein are understood to encompass the plural as well as the singular.

Unless otherwise indicated, the citation of any document is not to be construed as an admission that it is prior art with response to the present invention.

All percentages listed in this specification are percentages of solids/active amounts by weight (wt%), unless otherwise specifically mentioned.

The present invention relates to a composition comprising an orally-acceptable carrier and an anionic copolymer of N-vinyl pyrrolidone with an anionic monomer compound containing phosphorus used to deliver mineralization and anti-erosion benefits in oral care formulations.

The anionic copolymers used in the present invention are described by the formula I:

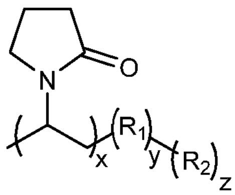

(I)

- $R_1$ is derived from methacryloxyethyl phosphate; and
- $R_2$ is a repeat unit to improve co-polymerization and solubility selected from hydroxyethyl acrylate (HEA), 2-hydroxypropyl acrylate, 4-hydroxybutyl acrylate, 2-Hydroxyethyl methacrylate, Hydroxypolyethoxy allyl ether, 3-Phenoxy 2 hydroxy propyl methacrylate, glycerol monomethyacrylate and N-(2-Hydroxypropyl)methacrylamide.

Wherein "x" represents the number of VP units and is an integer; "y" represents the number of $R_1$ units and is an integer; "z" represents the number of $R_2$ units and is an integer; and the ratio of "x" to "y+z" is from about 1:99 to about 99:1; and wherein the composition further comprises fluoride.

The anionic copolymers of vinyl pyrrolidone (Anionic PVP-copolymers) as defined in claim 1 are prepared via copolymerization of N-vinyl pyrrolidone (VP) monomers with anionic monomers containing phosphorus. Anionic monomers containing phosphorus include monomers comprising phosphonate groups, such as, for example, vinyl phosphonic acid (VPA), dimethyl vinylphosphonate, allylphosphonic acid, diethyl allylphosphate, diethyl vinylphosphonate, as well as, monomer compounds comprising phosphate groups, such as, for example, methacryloxyethyl phosphate (MOEP), Bis(2-methacryloxyethyl) phosphate, allyl phosphate compounds, and combinations of two or more thereof. Anionic copolymers of vinyl pyrrolidone (Anionic PVP-copolymers) may be prepared via copolymerization of N-vinyl pyrrolidone (VP) monomers with anionic monomers comprising phosphonate groups such as vinyl phosphonic acid. According to the present invention, the anionic copolymers of vinyl pyrrolidone (Anionic PVP-copolymers) are prepared via copolymerization of N-vinyl pyrrolidone (VP) monomers with the anionic monomer comprising phosphate groups methacryloxyethyl phosphate (MOEP). As will be understood by those of skill in the art, with respect to Formula I, the repeat unit $R_1$ may be described as being derived from an anionic monomer containing phosphorus as described herein. According to the present invention, $R_1$ is a repeat unit derived from methacryloxyethyl phosphate (MOEP).

Also disclosed but not claimed are monomers which can be modified post-polymerization to display a phosphonate or a phosphate functional group, such as maleic anhydride followed by a condensation reaction with an alcohol displaying the phosphate functional group, for example 2-hydroxyethyl dihydrogen phosphate.

Some of monomers and the post-polymerization modifications may result in displaying both a positive and negative charge, or zwitterionic.

The polymers of Formula I include repeat units $R_2$ which are derived from monomer compounds used to increase co-polymerization and/or solubility. The monomer compounds are generally selected based on their hydrophilicity and

include monomer compounds having an alcohol functional group. Monomer compounds according to the present invenition for use to achieve $R_2$ repeat units are hydroxyethyl acrylate (HEA), 2-hydroxypropyl acrylate, 4-hydroxybutyl acrylate, 2-Hydroxyethyl methacrylate, Hydroxypolyethoxy allyl ether, 3-Phenoxy 2 hydroxy propyl methacrylate, glycerol monomethyacrylate, N-(2-Hydroxypropyl)methacrylamide.

The anionic copolymers for use in the present invention may be heterogenous in the distribution of the anionic monomers containing phosphorus and can vary in the percent by weight of each repeat unit and ratio of the respective monomers/repeat units. For example, in certain embodiments, the repeat units derived from vinyl pyrrolidone and anionic monomers containing phosphorus ($R_1$) are independently from about 5 to about 95% by weight of the polymer, including from about 10 to about 90%, 20 to about 80%, 20 to about 50%, 30 to about 50%, 30 to about 40%, 50 to about 90%, 50 to about 70%, 50 to about 60%, 60 to about 70% by weight of the polymer. In certain preferred embodiments, the repeat units derived from vinyl pyrrolidone is from about 10 to about 40 %, including about 30 weight percent, by weight of the polymer, or from about 50 to about 70%, including about 60%, by weight of the polymer.

In certain preferred embodiments, z is zero. In certain other embodiments, wherein z is non-zero, the $R_2$ repeat units are from about 1 to about 40 %, including from about 5 to about 20%, or about 10 to about 20% of $R_2$ repeat units by weight of polymer.

The ratio of repeat units derived from vinyl pyrrolidone to repeat units derived from anionic monomers containing phosphorus ($R_1$) may be from about 10:90 to about 90:10, including from about 20:80 to about 80:20, about 30:70 to about 70:30, about 40:60 to about 60:40, and about 50:50. In certain preferred embodiments, the ratio of repeat units derived from vinyl pyrrolidone to repeat units derived from anionic monomers containing phosphorus ($R_1$) is from about 50:50 to about 70:30, including about 60:30 to about 70:30. In certain preferred embodiments, the ratio of repeat units derived from vinyl pyrrolidone to repeat units derived from anionic monomers containing phosphorus ($R_1$) is from about 30:70 to about 50:50, including about 30:50 to about 30:60.

The copolymers of the present invention may be of any suitable molecular weight. In certain embodiments, the polymers have a weight average molecular weight of from about 10,000 to about 1,000,000 including from about 10,000 to about 750,000, about 10,000 to about 500,000, about 10,000 to about 250,000, and about 150,000. In certain preferred embodiments, the average molecular weight is from about 10,000 to about 500,000, including about 10,000 to about 250,000. In certain preferred embodiments, the average molecular weight is from about 10,000 to about 250,000, including about 150,000.

The copolymers may comprise cross-linking agents. Cross-linking agents suitable for use in the present invention comprise at least two reactive sites which are electrophiles designed to react easily with phosphates or phosphonates. When the cross-linker has two reactive sites it is bifunctional and can thus react with two phosphate/phosphonate groups e.g. two anionic units in different polymer chains. The distance between the reactive groups may be increased by a spacer moiety. This spacer is often an aliphatic chain or a polyether construct like poly- or oligoethylene glycols. Preferably the cross-linking agent is bi-, tri- or tetrafunctional, although bi- or trifunctional is preferred and bifunctional is most preferred. Suitable cross-linking agents are known and include organic cationic polymers such as polyquaternium and polyethyleneimine, chitosan, polyvalent cations. Preferred cross-linking molecules are divalent cations such as calcium, zinc, tin, magnesium, manganese and their acetates, nitrates, phosphates, carbonates, tartrates, malonates, propionates, benzoates, or citrates thereof, and the like. Most preferred cross-linking molecules are calcium salts. Other suitable cross-linking agents may include, but it is not limited to, positively charged amino acids, peptides or proteins. These cross-linking agents may be used individually or in combination with each other. **In** general, it is preferred to have higher polymer concentrations and lower concentrations of cross-linking agent to achieve a composition of the desired nature. It is preferable to minimize the amount of cross-linker used. The molar ratio of cross-linking agent to polymer based on the number of functional groups in the cross-linking agent and the number of accessible phosphate/phosphonate groups in the polymer is preferably 0.2:1 or less, more preferably 0.1:1 or less and most preferably 0.05:1 or less. The molar ratio is based on the number of groups available for cross-linking on the cross-linker and on the polymer. For the cross-linker it will depend on the functionality (bi-, tri-, tetrafunctional etc.) and on the polymer to the accessibility of the anionic groups.

The compositions of the present invention may comprise from about 0.01~10% of the anionic polymers of the present invention. **In** certain preferred embodiments, the compositions comprise from about 0.01 to about 5%, from about 0.01 to about 3%, from about 0.01 to about 2%, from about 0.01 to about 1%, or from about 0.01 to about 0.5% or from about 0.01 to about 0.05% by weight of the anionic polymer of Formula I. **In** certain preferred embodiments, the composition comprises from about 0.01 to about 1%, preferably from about 0.01 to about 0.5%, or from about 0.01 to about 0.1% by weight of the anionic polymer of Formula I.

Any of a wide variety of orally-acceptable vehicles may be used in the present compositions. The vehicle can be aqueous or non-aqueous. The aqueous vehicle is generally water, although water/alcohol mixtures may also be employed. **In** certain embodiments, water is added to q.s. (Quantum Sufficit, Latin for "as much as needed") the composition. In certain embodiments, the aqueous phase comprises from about 60% to about 95%, or from about 75% to about 90%, by weight of the composition. In certain compositions, water is present in an amount of from about 60%

to about 95%, or from about 75% to about 90%. Alternatively, the compositions of the present invention may be formulated in a dry powder, chewing gum, film, semi-solid, solid or liquid concentrate form. In such embodiments, for example, water is added to q.s. as necessary in the case of liquid concentrates or powdered formulations, or water may be removed using standard evaporation procedures known in the art to produce a composition in dry powder form. Evaporated, or freeze dried forms are advantageous for storage and shipping.

In some embodiments, alcohol may be added to the composition. Any of a variety of alcohols represented by the formula $R_3$-OH, wherein $R_3$ is an alkyl group having from 2 to 6 carbons, may be used in the present invention. Examples of suitable alcohols of formula $R_3$-OH include ethanol; n-propanol, iso-propanol; butanols; pentanols; hexanols, and combinations of two or more thereof, and the like. In certain embodiments, the alcohol is, or comprises, ethanol.

In some embodiments, the alcohol may be present in the composition in an amount of about 10.0% v/v or greater of the total composition, or from about 10.0% to about 35.0% v/v of the total composition, or from about 15.0% to about 30.0% v/v of the total composition and may be from about 20.0% to about 25.0% v/v of the total composition.

In some embodiments, the compositions may comprise a reduced level of alcohol. The phrase "reduced level" of alcohol means an amount of a $R_3$-OH alcohol of about 10% v/v or less, or about 5% v/v or less, or about 1.0% v/v or less, or about 0.1% v/v or less by volume of the total composition. In certain embodiments, the compositions of the present invention are free of $R_3$-OH alcohols.

The compositions of the present invention preferably have a pH of about 11 or less. In certain embodiments, the composition have a pH of from about 3 to less than 7, or from about 3.5 to less than 7, or from about 3.5 to about 6.5, or from about 3.5 to about 5.5, or from about 3.5 to about 5.0.

As will be recognized by those of skill in the art, the pH of the composition may be adjusted or achieved using a buffer in an amount effective to provide the composition with a pH below 11. The composition can optionally comprise at least one pH modifying agents among those useful herein include acidifying agents to lower pH, basifying agents to raise pH, and buffering agents to control pH within a desired range. For example, one or more compounds selected from acidifying, basifying and buffering agents can be included to provide a pH of about 2 to about 7, or in various embodiments from about 3 to about 6, or from about 4 to about 5. Any orally acceptable pH modifying agent can be used including without limitation carboxylic and sulfonic acids, acid salts (e.g., monosodium citrate, disodium citrate, monosodium malate, etc.), alkali metal hydroxides such as sodium hydroxide, borates, silicates, imidazole and mixtures thereof. One or more pH modifying agents are optionally present in a total amount effective to maintain the composition in an orally acceptable pH range. In certain embodiments, inorganic acids may be used as the buffer added to the composition.

In certain embodiments, organic acids may be used as the buffer added to the composition. Organic acids suitable for use in the compositions of the present invention include, but are not limited to, ascorbic acid, sorbic acid, citric acid, glycolic acid, lactic acid and acetic acid, benzoic acid, salicylic acid, phthalic acid, phenolsulphonic acid, and mixtures thereof, optionally, the organic acid is selected from the group consisting of benzoic acid, sorbic acid, citric acid and mixtures thereof, or optionally, the organic acid is benzoic acid.

In some embodiments, useful buffer systems have been found to be sodium benzoate/benzoic acid, sodium citrate/citric acid, phosphoric acid/sodium/potassium phosphate.

Generally the amount of buffer is between about 0.001% (or about 0.001% w/v) to about 5.0% (or about 5.0% w/v) of the composition. In certain embodiment, the buffer is present in amounts of from 0.001% (or about 0.001% w/v) to 1.0% w/v (or about 1.0% w/v) of the composition, or between about 0.100% (or about 0.100% w/v) to about 1.0% (or about 1.0% w/v) of the composition.

The compositions of the present invention may further comprise any of a variety of optional ingredients therein, including, but not limited to oily components, active ingredients, additional surfactants, humectants, solvents, flavors, sweeteners, colorants, preservatives, and the like.

Any of a variety of oily components may be used in the present compositions. The oily component may comprise any one or more oils, or other materials that are water insoluble, or substantially water-insoluble, meaning that its solubility is less than about 1% by weight in water at 25° C. or, optionally, less than about 0.1%. In certain embodiments, the oily component of the present invention comprises, consists essentially of, or consists of, at least one essential oil, i.e. a natural or synthetic (or combination thereof) concentrated hydrophobic material of vegetable origin, generally containing volatile compounds, at least one flavor oil, or a combination of two or more thereof. Examples of suitable essential oils, flavor oils, and their amounts are described below. In certain embodiments, the composition comprises a total amount of oily component of about 0.05% w/w or more, about 0.1% w/w or more, or about 0.2% w/w or more of oily component.

In certain embodiments, compositions of the present invention comprise essential oils. Essential oils are volatile aromatic oils which may be synthetic or may be derived from plants by distillation, expression or extraction, and which usually carry the odor or flavor of the plant from which they are obtained. Useful essential oils may provide antiseptic activity. Some of these essential oils also act as flavoring agents. Useful essential oils include but are not limited to citra, thymol, menthol, methyl salicylate (wintergreen oil), eucalyptol, carvacrol, camphor, anethole, carvone, eugenol, isoeugenol, limonene, osimen, n-decyl alcohol, citronel, a-salpineol, methyl acetate, citronellyl acetate, methyl eugenol, cineol, linalool, ethyl linalaol, safrola vanillin, spearmint oil, peppermint oil, lemon oil, orange oil, sage oil, rosemary oil,

cinnamon oil, pimento oil, laurel oil, cedar leaf oil, gerianol, verbenone, anise oil, bay oil, benzaldehyde, bergamot oil, bitter almond, chlorothymol, cinnamic aldehyde, citronella oil, clove oil, coal tar, eucalyptus oil, guaiacol, tropolone derivatives such as hinokitiol, avender oil, mustard oil, phenol, phenyl salicylate, pine oil, pine needle oil, sassafras oil, spike lavender oil, storax, thyme oil, tolu balsam, terpentine oil, clove oil, and combinations thereof.

In certain embodiments, the essential oils are selected from the group consisting of thymol ($(CH_3)_2CHC_6H_3(CH_3)OH$, also known as isopropyl-m-cresol), eucalyptol ($C_{10}H_{18}O$, also known as cineol), menthol ($CH_3C_6H_9(C_3H_7)OH$), also known as hexahydrothymol), methyl salicylate ($C_6H_4OHCOOCH_3$, also known as wintergreen oil), isomers of each of these compounds, and combinations of two or more thereof. In some embodiments, the compositions of the present invention contain thymol. In some embodiments, the compositions of the present invention contain menthol. In some embodiments, the composition contains all four of these essential oils.

In certain embodiments, thymol is employed in amounts of from about 0.0001% to about 0.6% w/v, or from about 0.005% to about 0.07% w/v of the composition. In certain embodiments, eucalyptol may be employed in amounts of from about 0.0001% to about 0.51% w/v, or from about 0.0085% to about 0.10% w/v of the composition. In certain embodiments, menthol is employed in amounts of from about 0.0001% to about 0.25% w/v, or from about 0.0035% to about 0.05% w/v of the composition. In certain embodiments, methy salicylate is employed in amounts of from about 0.0001% to about 0.28% w/v, or from about 0.004% to about 0.07% w/v of the composition. In certain embodiments, the total amount of all of such essential oils present in the disclosed compositions can be from about 0.0004% to about 1.64% w/v, or from about 0.0165% to about 0.49% w/v of the composition.

The compositions of the present invention further comprise fluoride. In certain embodiments, fluoride providing compounds may be present in the mouth rinse compositions of this invention. These compounds may be slightly water soluble or may be fully water soluble and are characterized by their ability to release fluoride ions or fluoride containing ions in water. Typical fluoride providing compounds are inorganic fluoride salts such as soluble alkali metal, alkaline earth metal, and heavy metal salts, for example, sodium fluoride, potassium fluoride, ammonium fluoride, cupric fluoride, zinc fluoride, stannic fluoride, stannous fluoride, barium fluoride, sodium hexafluorosilicate, ammonium hexafluorosilicate, sodium fluorozirconate, sodium monofluorophosphate, aluminum mono- and difluorophosphate and fluorinated sodium calcium pyrophosphate. Amine fluorides, such as N'-octadecyltrimethylendiamine-N,N,N'-tris(2-ethanol)-dihydrofluoride and 9-octadecenylamine-hydrofluoride), may also be used. In certain embodiments, the fluoride providing compound is generally present in an amount sufficient to release up to about 5%, or from about 0.001% to about 2%, or from about 0.005% to about 1.5% fluoride by weight of the composition.

In certain embodiments, sensitivity reducing agents, such as potassium salts of nitrate and oxalate in an amount from about 0.1% to about 5.0% w/v of the composition may be incorporated into the present invention. Other potassium releasing compounds are feasible (e.g. KCl). High concentrations of calcium phosphates may also provide some added sensitivity relief. These agents are believed to work by either forming an occlusive surface mineral deposit on the tooth surface or through providing potassium to the nerves within the teeth to depolarize the nerves. A more detailed discussion of suitable sensitivity reducing agents can be found in US 2006/0013778 to Hodosh and U.S. Pat. No. 6,416,745 to Markowitz et al..

In certain embodiments, compounds with anti-calculus benefits (e.g. various carboxylates, polyaspartic acid, etc.) may be incorporated into the present invention. Also useful as an anticalculus agent are the anionic polymeric polycarboxylates. Such materials are well known in the art, being employed in the form of their free acids or partially or preferably fully neutralized water soluble alkali metal (e.g. potassium and preferably sodium) or ammonium salts. Preferred are 1:4 to 4:1 by weight copolymers of maleic anhydride or acid with another polymerizable ethylenically unsaturated monomer, preferably methyl vinyl ether (methoxyethylene) having a molecular weight (M.W.) of about 30,000 to about 1,000,000. These copolymers are available, for example, as Gantrez 25 AN 139 (M.W. 500,000), AN 119 (M.W. 250,000) and preferably S-97 Pharmaceutical Grade (M.W. 70,000), of GAF Chemicals Corporation.

Additional anti-calculus agents may be selected from the group consisting of polyphosphates (including pyrophosphates) and salts thereof polyamino propane sulfonic acid (AMPS) and salts thereof; polyolefin sulfonates and salts thereof; polyvinyl phosphates and salts thereof; polyolefin phosphates and salts thereof; diphosphonates and salts thereof; phosphonoalkane carboxylic acid and salts thereof; polyphosphonates and salts thereof; polyvinyl phosphonates and salts thereof; polyolefin phosphonates and salts thereof; polypeptides; and mixtures thereof; carboxy-substituted polymers; and mixtures thereof. In one embodiment, the salts are alkali metal or ammonium salts. Polyphosphates are generally employed as their wholly or partially neutralized water-soluble alkali metal salts such as potassium, sodium, ammonium salts, and mixtures thereof. The inorganic polyphosphate salts include alkali metal (e.g. sodium) tripolyphosphate, tetrapolyphosphate, dialkyl metal (e.g. disodium) diacid, trialkyl metal (e.g. trisodium) monoacid, potassium hydrogen phosphate, sodium hydrogen phosphate, and alkali metal (e.g. sodium) hexametaphosphate, and mixtures thereof. Polyphosphates larger than tetrapolyphosphate usually occur as amorphous glassy materials. In one embodiment the polyphosphates are those manufactured by FMC Corporation, which are commercially known as Sodaphos ($n \approx 6$), Hexaphos ($n \approx 13$), and Glass H ($n \approx 21$, sodium hexametaphosphate), and mixtures thereof. The pyrophosphate salts useful in the present invention include, alkali metal pyrophosphates, di-, tri-, and monopotassium

or sodium pyrophosphates, dialkali metal pyrophosphate salts, tetraalkali metal pyrophosphate salts, and mixtures thereof. In one embodiment the pyrophosphate salt is selected from the group consisting of trisodium pyrophosphate, disodium dihydrogen pyrophosphate ($Na_2H_2P_2O_7$), dipotassium pyrophosphate, tetrasodium pyrophosphate ($Na_4P_2O_7$), tetrapotassium pyrophosphate ($K_4P_2O_7$), and mixtures thereof. Polyolefin sulfonates include those wherein the olefin group contains 2 or more carbon atoms, and salts thereof. Polyolefin phosphonates include those wherein the olefin group contains 2 or more carbon atoms. Polyvinylphosphonates include polyvinylphosphonic acid. Diphosphonates and salts thereof include azocycloalkane-2,2-diphosphonic acids and salts thereof, ions of azocycloalkane-2,2-diphosphonic acids and salts thereof, azacyclohexane-2,2-diphosphonic acid, azacyclopentane-2,2-diphosphonic acid, N-methyl-azacyclopentane-2,3-diphosphonic acid, EHDP (ethane-1-hydroxy-1,1,-diphosphonic acid), AHP (azacycloheptane-2,2-diphosphonic acid), ethane-1-amino-1,1-diphosphonate, dichloromethane-diphosphonate, etc. Phosphonoalkane carboxylic acid or their alkali metal salts include PPTA (phosphonopropane tricarboxylic acid), PBTA (phosphonobutane-1,2,4-tricarboxylic acid), each as acid or alkali metal salts. Polyolefin phosphates include those wherein the olefin group contains 2 or more carbon atoms. Polypeptides include polyaspartic and polyglutamic acids.

In certain embodiments, zinc salts such as zinc chloride, zinc acetate or zinc citrate may be added as an astringent for an "antiseptic cleaning" feeling, as a breath protection enhancer or as anti-calculus agent in an amount of from about 0.0025% w/v to about 0.75% w/v of the composition.

Any of a variety of additional surfactants may be used in the present invention. Suitable surfactants may include anionic, non-ionic, cationic, amphoteric, zwitterionic surfactants, and combinations of two or more thereof. Examples of suitable surfactants are disclosed, for example, in U.S. Pat. No. 7,417,020 to Fevola, et al.

In certain embodiments, the compositions of the present invention comprise a non-ionic surfactant. Those of skill in the art will recognize that any of a variety of one or more non-ionic surfactants include, but are not limited to, compounds produced by the condensation of alkylene oxide groups (hydrophilic in nature) with an organic hydrophobic compound which may be aliphatic or alkyl-aromatic in nature. Examples of suitable nonionic surfactants include, but are not limited to, alkyl polyglucosides; alkyl glucose amines, block copolymers such as ethylene oxide and propylene oxide copolymers e.g. Poloxamers; ethoxylated hydrogenated castor oils available commercially for example under the trade name CRODURET (Croda Inc., Edison, N.J.); alkyl polyethylene oxide e.g. Polysorbates, and/or; fatty alcohol ethoxylates; polyethylene oxide condensates of alkyl phenols; products derived from the condensation of ethylene oxide with the reaction product of propylene oxide and ethylene diamine; ethylene oxide condensates of aliphatic alcohols; long chain tertiary amine oxides; long chain tertiary phosphine oxides; long chain dialkyl sulfoxides; and mixtures thereof.

Exemplary non-ionic surfactants are selected from the group known as poly(oxyethylene)-poly(oxypropylene) block copolymers. Such copolymers are known commercially as poloxamers and are produced in a wide range of structures and molecular weights with varying contents of ethylene oxide. These non-ionic poloxamers are non-toxic and acceptable as direct food additives. They are stable and readily dispersible in aqueous systems and are compatible with a wide variety of formulations and other ingredients for oral preparations. These surfactants should have an HLB (Hydrophilic-Lipophilic Balance) of between about 10 and about 30 and preferably between about 10 and about 25. By way of example, non-ionic surfactants useful in this invention include the poloxamers identified as poloxamers 105, 108, 124, 184, 185, 188, 215, 217, 234, 235, 237, 238, 284, 288, 333, 334, 335, 338, 407, and combinations of two or more thereof In certain preferred embodiments, the composition comprises poloxamer 407.

In certain embodiments, the compositions of the claimed invention comprise less than about 9% of non-ionic surfactant, less than 5%, or less than 1.5%, or less than 1%, or less than 0.8, less than 0.5%, less than 0.4%, or less than .3% of non-ionic surfactants. In certain embodiments, the composition of the present invention is free of non-ionic surfactants.

In certain embodiments, the compositions of the present invention also contain at least one alkyl sulfate surfactant. In certain embodiments, suitable alkyl sulfate surfactants include, but are not limited to sulfated $C_8$ to $C_{18}$, optionally sulfated $C_{10}$ to $C_{16}$ even numbered carbon chain length alcohols neutralized with a suitable basic salt such as sodium carbonate or sodium hydroxide and mixtures thereof such that the alkyl sulfate surfactant has an even numbered $C_8$ to $C_{18}$, optionally $C_{10}$ to $C_{16}$, chain length. In certain embodiments, the alkyl sulfate is selected from the group consisting of sodium lauryl sulfate, hexadecyl sulfate and mixtures thereof. In certain embodiments, commercially available mixtures of alkyl sulfates are used. A typical percentage breakdown of alkyl sulfates by alkyl chain length in commercially available sodium lauryl sulfate (SLS) is as follows:

| Alkyl Chain Length | Component Percentage in SLS |
|---|---|
| $C_{12}$ | >60% |

$C_{14}$ 20%-35%

$C_{16}$ <10%

$C_{10}$ <1%

$C_{18}$ <1%

**In** certain embodiments, the alkyl sulfate surfactant is present in the composition from about 0.001% to about 6.0% w/v, or optionally from about 0.1% to about 0.5% w/v of the composition.

Another suitable surfactant is one selected from the group consisting of sarcosinate surfactants, isethionate surfactants and taurate surfactants. Preferred for use herein are alkali metal or ammonium salts of these surfactants, such as the sodium and potassium salts of the following: lauroyl sarcosinate, myristoyl sarcosinate, palmitoyl sarcosinate, stearoyl sarcosinate and oleoyl sarcosinate. The sarcosinate surfactant may be present in the compositions of the present invention from about 0.1% to about 2.5%, or from about 0.5% to about 2% by weight of the total composition.

Zwitterionic synthetic surfactants useful in the present invention include derivatives of aliphatic quaternary ammonium, phosphonium, and sulfonium compounds, in which the aliphatic radicals can be straight chain or branched, and wherein one of the aliphatic substituents contains from about 8 to 18 carbon atoms and one contains an anionic water-solubilizing group, e.g., carboxy, sulfonate, sulfate, phosphate or phosphonate.

The amphoteric surfactants useful in the present invention include, but are not limited to, derivatives of aliphatic secondary and tertiary amines in which the aliphatic radical can be a straight chain or branched and wherein one of the aliphatic substituents contains from about 8 to about 18 carbon atoms and one contains an anionic water-solubilizing group, e.g., carboxylate, sulfonate, sulfate, phosphate, or phosphonate. Examples of suitable amphoteric surfactants include, but are not limited alkylimino-diproprionates, alky lamphoglycinates (mono or di), alkylamphoproprionates (mono or di), alkylamphoacetates (mono or di), N-alkyl [3-aminoproprionic acids, alkylpolyamino carboxylates, phosphorylated imidazolines, alkyl betaines, alkylamido betaines, alkylamidopropyl betaines, alkyl sultaines, alkylamido sultaines, and mixtures thereof. **In** certain embodiments, the amphoteric surfactant is selected from the group consisting of alkylamidopropyl betaines, amphoacetates such as sodium auroamphoacetate and mixtures thereof. Mixtures of any of the above mentioned surfactants can also be employed. A more detailed discussion of anionic, nonionic and amphoteric surfactants can be found in U.S. Pat. No. 7,087,650 to Lennon; U.S. Pat. No. 7,084,104 to Martin et al.; U.S. Pat. No. 5,190,747 to Sekiguchi et al.; and U.S. Pat. No. 4,051,234, Gieske, et al..

**In** certain embodiments, the compositions of the claimed invention comprise less than about 9% of amphoteric surfactant, less than 5%, or less than 1.5%, or less than 1%, or less than 0.8, less than 0.5%, less than 0.4%, or less than .3% of amphoteric surfactants. **In** certain embodiments, the composition of the present invention is free of amphoteric surfactants.

Additional surfactants may be added with the alkyl sulfate surfactant to aid in solubilization of the essential oils provided such surfactants do not affect the bioavailability of the essential oils. Suitable examples include additional anionic surfactants, nonionic surfactants, amphoteric surfactants and mixtures thereof. However, in certain embodiments, the total surfactant concentration (including the alkyl sulfate surfactant alone or in combination with other surfactants) for mouth rinses of the present invention should not exceed or should about 9% or less, optionally, the total surfactant concentration should be about 5% or less, optionally about 1% or less, optionally about 0.5% or less w/w% of active surfactant by weight of the composition.

In certain embodiments, a sugar alcohol (humectant) is also added to the oral compositions of the present invention. The sugar alcohol solvent(s) may be selected from those multi-hydroxy-functional compounds that are conventionally used in oral and ingestible products. In certain embodiments, the sugar alcohol (s) should be nonmetabolized and non-fermentable sugar alcohol (s). In specific embodiments, the sugar alcohols include, but are not limited to sorbitol, glycerol, xylitol, mannitol, maltitol, inositol, allitol, altritol, dulcitol, galactitol, glucitol, hexitol, iditol, pentitol, ribitol, erythritol and mixtures thereof. Optionally, the sugar alcohol is selected from the group consisting of sorbitol and xylitol or mixtures thereof. In some embodiments, the sugar alcohol is sorbitol. In certain embodiments, the total amount of sugar alcohol (s), which are added to effectively aid in the dispersion or dissolution of the mouth rinse or other ingredients, should not exceed about 50% w/ of the total composition. Or, total amount of sugar alcohol should not exceed about 30% w/v of the total composition. Or, total amount of sugar alcohol should not exceed 25% w/v of the total composition. The sugar alcohol can be in an amount of from about 1.0% to about 24% w/v, or from about 1.5% to about 22% w/v, or from about 2.5% to about 20% w/v of the total composition.

In certain embodiments, a polyol solvent is added to the composition. The polyol solvent comprises a polyol or polyhydric alcohol selected from the group consisting of polyhydric alkanes (such as propylene glycol, glycerin, butylene

glycol, hexylene glycol, 1,3-propanediol); polyhydric alkane esters (dipropylene glycol, ethoxydiglycol); polyalkene glycols (such as polyethylene glycol, polypropylene glycol) and mixtures thereof. In certain embodiments, the polyol solvent can be present in an amount of from 0% to about 40% w/v, or from about 0.5% to about 20% w/v, or from about 1.0% to about 10% w/v of the composition.

Sweeteners such as aspartame, sodium saccharin (saccharin), sucralose, stevia, acesulfame K and the like may be added for better taste in amounts of from about 0.0001% w/v to about 1.0% w/v. In certain preferred embodiments, the sweetener comprises sucralose.

In certain embodiments, the composition further comprises flavors or flavorants to modify or magnify the taste of the composition, or reduce or mask the sharp "bite" or "burn" of ingredients such as thymol. Suitable flavors include, but are not limited to, flavor oils such as oil of anise, anethole, benzyl alcohol, spearmint oil, citrus oils, vanillin and the like may be incorporated. Other flavors such as citrus oils, vanillin and the like may be incorporated to provide further taste variations. In these embodiments, the amount of flavor oil added to the composition can be from about 0.001% to about 5% w/v, or from about 0.01% to about 0.3% w/v of the total composition. The particular flavors or flavorants, and other taste improving ingredients, employed will vary depending upon the particular taste and feel desired. Those skilled in the art can select and customize these types of ingredients to provide the desired results.

In certain embodiments, acceptably approved food dyes may be used to provide a pleasing color to the compositions of the invention. These may be selected from, but not limited to, the long list of acceptable food dyes. Suitable dyes for this purpose include FD&C yellow #5, FD&C yellow #10, FD&C blue #1 and FD&C green #3. These are added in conventional amounts, typically in individual amounts of from about 0.00001% w/v to about 0.0008% w/v, or from about 0.000035% w/v to about 0.0005% w/v of the composition.

Other conventional ingredients may be used in the liquid or mouth rinse compositions of this invention, including those known and used in the art. Examples of such ingredients include thickeners, suspending agents and softeners. Thickeners and suspending agents useful in the compositions of the present invention can be found in U.S. Pat. No. 5,328,682 to Pullen et al.. In certain embodiments, these are incorporated in amounts of from about 0.1% w/v to about 0.6% w/v, or about 0.5% w/v of the composition.

In some embodiments, antimicrobial preservatives may be added to the composition. Some antimicrobial preservatives which may be used include , but are not limited to cationic antibacterials, such as sodium benzoate, polyquaternium polycationic polymers (i.e polyquaternium-42: Poly[oxyethylene(dimethylimino)ethylene (dimethylimino) ethylene dichloride]), quaternary ammonium salts or quaternary ammonium compounds, parabens (i.e. parahydroxybenzoates or esters of parahydroxybenzoic acid), hydroxyacetophenone, 1,2-Hexanediol, Caprylyl Glycol, chlorhexidine, alexidine, hexetidine, benzalkonium chloride, domiphen bromide, cetylpyridinium chloride (CPC), tetradecylpyridinium chloride (TPC), N-tetradecyl-4-ethylpyridinium chloride (TDEPC), octenidine, bisbiguanides, zinc or stannous ion agents, grapefruit extract, and mixtures thereof. Other antibacterial and antimicrobial agents include, but are not limited to: 5-chloro-2-(2,4-dichlorophenoxy)-phenol, commonly referred to as triclosan; 8-hydroxyquinoline and its salts, copper II compounds, including, but not limited to, copper(II) chloride, copper(II) sulfate, copper(II) acetate, copper(II) fluoride and copper(II) hydroxide; phthalic acid and its salts including, but not limited to those disclosed in U.S. Pat. No. 4,994,262, including magnesium monopotassium phthalate; sanguinarine; salicylanilide; iodine; sulfonamides; phenolics; delmopinol, octapinol, and other piperidino derivatives; niacin preparations; nystatin; apple extract; thyme oil; thymol; antibiotics such as augmentin, amoxicillin, tetracycline, doxycycline, minocycline, metronidazole, neomycin, kanamycin, cetylpyridinium chloride, and clindamycin; analogs and salts of the above; methyl salicylate; hydrogen peroxide; metal salts of chlorite; pyrrolidone ethyl cocoyl arginate; lauroyl ethyl arginate monochlorohydrate; and mixtures of all of the above. In another embodiment, the composition comprises phenolic antimicrobial compounds and mixtures thereof. Antimicrobial components may be present from about 0.001% to about 20% by weight of the oral care composition. In another embodiment the antimicrobial agents generally comprise from about 0.1% to about 5% by weight of the oral care compositions of the present invention.

Other antibacterial agents may be basic amino acids and salts. Other embodiments may comprise arginine.

In certain embodiments, the compositions may include whitening agents, oxidizing agents, anti-inflammatories, chelating agents, abrasives, combinations thereof, and the like.

A whitening agent may be included as an active in the present compositions. The actives suitable for whitening are selected from the group consisting of alkali metal and alkaline earth metal peroxides, metal chlorites, polyphosphates, perborates inclusive of mono and tetrahydrates, perphosphates, percarbonates, peroxyacids, and persulfates, such as ammonium, potassium, sodium and lithium persulfates, and combinations thereof. Suitable peroxide compounds include hydrogen peroxide, urea peroxide, calcium peroxide, carbamide peroxide, magnesium peroxide, zinc peroxide, strontium peroxide and mixtures thereof. In one embodiment the peroxide compound is carbamide peroxide. Suitable metal chlorites include calcium chlorite, barium chlorite, magnesium chlorite, lithium chlorite, sodium chlorite, and potassium chlorite. Additional whitening actives may be hypochlorite and chlorine dioxide. In one embodiment the chlorite is sodium chlorite. In another embodiment the percarbonate is sodium percarbonate. In one embodiment the persulfates are oxones. The level of these substances is dependent on the available oxygen or chlorine, respectively, that the molecule is capable of

providing to bleach the stain. In one embodiment the whitening agents may be present at levels from about 0.01% to about 40%, in another embodiment from about 0.1% to about 20%, in another embodiment form about 0.5% to about 10%, and in another embodiment from about 4% to about 7%, by weight of the oral care composition.

The compositions of the invention may contain an oxidizing agent, such as a peroxide source. A peroxide source may comprise hydrogen peroxide, calcium peroxide, carbamide peroxide, or mixtures thereof. In some embodiments, the peroxide source is hydrogen peroxide. Other peroxide actives can include those that produce hydrogen peroxide when mixed with water, such as percarbonates, e.g., sodium percarbonates. In certain embodiments, the peroxide source may be in the same phase as a stannous ion source. In some embodiments, the composition comprises from about 0.01% to about 20% of a peroxide source, in other embodiments from about 0.1% to about 5%, in certain embodiments from about 0.2% to about 3%, and in another embodiment from about 0.3% to about 2.0% of a peroxide source, by weight of the oral composition. The peroxide source may be provided as free ions, salts, complexed, or encapsulated. It is desirable that the peroxide in the composition is stable. The peroxide may provide a reduction in staining, as measured by the Cycling Stain Test, or other relevant methods.

Anti-inflammatory agents can also be present in the compositions of the present invention. Such agents may include, but are not limited to, non-steroidal anti-inflammatory (NSAID) agents, oxicams, salicylates, propionic acids, acetic acids and fenamates. Such NSAIDs include but are not limited to ketorolac, flurbiprofen, ibuprofen, naproxen, indomethacin, diclofenac, etodolac, indomethacin, sulindac, tolmetin, ketoprofen, fenoprofen, piroxicam, nabumetone, aspirin, diflunisal, meclofenamate, mefenamic acid, oxyphenbutazone, phenylbutazone and acetaminophen. Use of NSAIDs such as ketorolac are claimed in U.S. Pat. No. 5,626,838. Disclosed therein are methods of preventing and/or treating primary and reoccurring squamous cell carcinoma of the oral cavity or oropharynx by topical administration to the oral cavity or oropharynx of an effective amount of an NSAID. Suitable steroidal anti-inflammatory agents include corticosteroids, such as fluccinolone, and hydrocortisone.

The present compositions may optionally contain chelating agents, also called chelants or sequestrants, many of which also have anticalculus activity or tooth substantive activity. Use of chelating agents in oral care products is advantageous for their ability to complex calcium such as found in the cell walls of bacteria. Chelating agents can also disrupt plaque by removing calcium from the calcium bridges which help hold this biomass intact. Chelating agents also have the ability to complex with metallic ions and thus aid in preventing their adverse effects on the stability or appearance of products. Chelation of ions, such as iron or copper, helps retard oxidative deterioration of finished products. In addition, chelants can in principle remove stains by binding to teeth surfaces thereby displacing color bodies or chromagens. The retention of these chelants can also prevent stains from accruing due to disruption of binding sites of color bodies on tooth surfaces. Therefore, chelants can aid in helping to mitigate stain and improve cleaning. A chelant may help to improve the cleaning as fused silica and abrasives clean in a mechanical mechanism while the chelant may help to provide chemical cleaning. Because the fused silica is a good mechanical cleaner, there may be more stain removed so a chelant may be desired to hold, suspend, or complex with the stain so it is not able to restain the tooth surface. Additionally, the chelant may coat the surface of the tooth to help prevent new stain. Chelants may be desired to be added to formulations containing cationic antibacterial agents. It may be desired to add chelants to stannous containing formulations. The chelant is able to help stabilize the stannous and keep a higher amount of the stannous bioavailable. The chelant may be used in stannous formulations which have a pH above about 4.0. In some formulations, the stannous may be stable without the need for a chelant as the stannous is more stable with fused silica as compared to precipitated silica.

Suitable chelating agents include soluble phosphate compounds, such as phytates and linear polyphosphates having two or more phosphate groups, including tripolyphosphate, tetrapolyphosphate and hexametaphosphate, among others. Preferred polyphosphates are those having the number of phosphate groups n averaging from about 6 to about 21, such as those commercially known as Sodaphos ($n \approx 6$), Hexaphos ($n \approx 13$), and Glass H ($n \approx 21$). Other polyphosphorylated compounds may be used in addition to or instead of the polyphosphate, in particular polyphosphorylated inositol compounds such as phytic acid, myo-inositol pentakis(dihydrogen phosphate); myo-inositol tetrakis(dihydrogen phosphate), myo-inositol trikis(dihydrogen phosphate), and an alkali metal, alkaline earth metal or ammonium salt thereof. Preferred herein is phytic acid, also known as myo-inositol 1,2,3,4,5,6-hexakis (dihydrogen phosphate) or inositol hexaphosphoric acid, and its alkali metal, alkaline earth metal or ammonium salts. Herein, the term "phytate" includes phytic acid and its salts as well as the other polyphosphorylated inositol compounds. The amount of chelating agent in the compositions will depend on the chelating agent used and typically will be from at least about 0.1% to about 20%, preferably from about 0.5% to about 10% and more preferably from about 1.0% to about 7%.

Still other phosphate compounds that are useful herein for their ability to bind, solubilize and transport calcium are the surface active organophosphate compounds described above useful as tooth substantive agents including organic phosphate mono-, di- or triesters.

Other suitable agents with chelating properties for use in controlling plaque, calculus and stain include polyphosphonates described in U.S. Pat. No. 3,678,154 to Widder et al., U.S. Pat. No. 5,338,537 to White, Jr.; carbonyl diphosphonates; acrylic acid polymer or copolymer in U.S. Pat. No. 4,847,070, Jul. 11, 1989 to Pyrz et al. and in U.S. Pat. No. 4,661,341, Apr. 28, 1987 to Benedict et al.; sodium alginate in U.S. Pat. No. 4,775,525, issued Oct. 4, 1988, to

Pera; polyvinyl pyrrolidone in GB 741,315, WO 99/12517 and U.S. Pat. No. 5,538,714 to Pink et al.; and copolymers of vinyl pyrrolidone with carboxylates in U.S. Pat. No. 5,670,138 to Venema et al.

Still other chelating agents suitable for use in the present invention are the anionic polymeric polycarboxylates. Such materials are well known in the art, being employed in the form of their free acids or partially or preferably fully neutralized water soluble alkali metal (e.g. potassium and preferably sodium) or ammonium salts. Examples are 1:4 to 4:1 copolymers of maleic anhydride or acid with another polymerizable ethylenically unsaturated monomer, preferably methyl vinyl ether (methoxyethylene) having a molecular weight (M.W.) of about 30,000 to about 1,000,000. These copolymers are available for example as Gantrez® AN 139 (M.W. 500,000), AN 119 (M.W. 250,000) and S-97 Pharmaceutical Grade (M.W. 70,000), of GAF Chemicals Corporation. Other operative polymeric polycarboxylates include the 1:1 copolymers of maleic anhydride with ethyl acrylate, hydroxyethyl methacrylate, N-vinyl-2-pyrrolidone, or ethylene, the latter being available for example as Monsanto EMA No. 1103, M.W. 10,000 and EMA Grade 61, and 1:1 copolymers of acrylic acid with methyl or hydroxyethyl methacrylate, methyl or ethyl acrylate, isobutyl vinyl ether or N-vinyl-2-pyrrolidone. Additional operative polymeric polycarboxylates are disclosed in U.S. Pat. No. 4,138,477, Feb. 6, 1979 to Gaffar and U.S. Pat. No. 4,183,914, Jan. 15, 1980 to Gaffar et al. and include copolymers of maleic anhydride with styrene, isobutylene or ethyl vinyl ether; polyacrylic, polyitaconic and polymaleic acids; and sulfoacrylic oligomers of M.W. as low as 1,000 available as Uniroyal ND-2. Other suitable chelants include polycarboxylic acids and salts thereof described in U.S. Pat. No. 5,015,467to Smitherman U.S. Pat. Nos. 5,849,271 and 5,622,689 both to Lukacovic; such as tartaric acid, citric acid, gluconic acid, malic acid; succinic acid, disuccinic acid and salts thereof, such as sodium or potassium gluconate and citrate; citric acid/alkali metal citrate combination; disodium tartrate; dipotassium tartrate; sodium potassium tartrate; sodium hydrogen tartrate; potassium hydrogen tartrate; acid or salt form of sodium tartrate monosuccinate, potassium tartrate disuccinate, and mixtures thereof. In some embodiments, there may be mixtures or combinations of chelating agents.

Suitable abrasives for use in the present invention may include, but are not limited to: perlite, silica such as sand or quartz, ground glass, silicon carbide, ilmenite ($FeTiO_3$), zircon oxide, zircon silicate, topaz, TiOz, precipitated lime, chalk, flour of pumice, zeolites, talcum, kaolin, kieselguhr, aluminum oxide, silicates, zinc orthophosphate, sodium bicarbonate (baking soda), plastic particles, alumina, hydrated alumina, calcium carbonate, calcium pyrophosphate, and mixtures thereof. The silica abrasive may be natural amorphous silica including diatomaceous earth; or a synthetic amorphous silica such as a precipitated silica; or a silica gel, such as a silica xerogel; or mixtures thereof.

Generally, an amount of abrasive suitable for use in the composition of the invention will be empirically determined to provide an acceptable level of cleaning and polishing, in accordance with the techniques well known in the art. In one embodiment, a composition of the present invention includes an abrasive. In one embodiment, a composition includes a silica abrasive. In one embodiment, a silica abrasive is present in an amount of from 0.001 wt. % to 30 wt. %. In one embodiment, a silica abrasive is present in an amount of from 1 wt. % to 15 wt. %. In one embodiment, a silica abrasive is present in an amount of from 4 wt. % to 10 wt. %

Other useful oral care actives and/or inactive ingredients and further examples thereof can be found in U.S. Pat. No. 6,682,722 to Majeti et al. and U.S. Pat. No. 6,121,315 to Nair et al..

The compositions of the present invention may be made according to any of a variety of methods disclosed herein and known in the art. In general, the described compositions may be prepared by combining the desired components in a suitable container and mixing them under ambient conditions in any conventional mixing means well known in the art, such as a mechanically stirred propeller, paddle, and the like.

The compounds and compositions of the present invention may be used in the oral cavity. According to certain embodiments, the compositions of the present invention can be used in methods of inhibiting, preventing, and or treateing the demineralization of teeth by contacting the tooth surface with a composition of the present invention. In certain embodiments, the compositions of the present invention can be used in methods of preventing or inhibiting the demineralization of teeth by contacting the tooth surface with a composition of the present invention.

In each of the above methods, the composition of the claimed method may be introduced to the tooth surface via any of a variety of methods. In certain embodiments, the composition is introduced into the oral cavity and applied to the tooth surface by a user as a mouthwash or mouth rinse. In certain embodiments, the composition is introduced to the oral cavity and applied to the tooth surface as toothpaste on an article for cleaning the teeth, e.g. a toothbrush. The compositions of the present invention may be further introduced via the mouth and applied to the tooth surface as a gum, lozenge, dissolvable strip, or the like.

Furthermore, the contacting step of the methods of the present invention may comprise contacting the tooth surface with the composition for any suitable amount of time. In certain embodiments, the contacting step comprises contacting the surface for less than thirty seconds. In certain embodiments, the contacting step comprises contacting the surface with the composition for thirty seconds or more, for example, for about thirty seconds, for about forty seconds, for about one minute, or for greater than one minute.

**EXAMPLES**

**REFERENCE EXAMPLE 1**

An anionic copolymer of vinyl pyrrolidone (anionic PVP-copolymer) was prepared via copolymerization of N-vinyl pyrrolidone (VP) monomer with vinyl phosphonic acid (VPA) monomer via radical polymerization. The resultant copolymer is referred to as (PVP-PVPA).

Briefly, isopropyl alcohol (IPA, 125 g) was heated to approximately 80°C in a round bottom flask under nitrogen atmosphere and gentle agitation. A monomer/initiator solution consisting of IPA (125 g), VPA (75 g), VP (175 g) and VAZO 52 (1 g, Chemours Inc.) was added gradually over 120 minutes. The reaction temperature was allowed to rise during the polymerization to 83-84°C and maintained at this temperature for an additional 30 minutes before cooling to 77°C. An initiator chase solution consisting of VAZO 52 (0.4 g) in IPA (5 g) was added and the reaction mass was maintained at 77°C for an additional hour. The polymer solution was then cooled, precipitated from hexane, and dried overnight under vacuum. The predicted composition of the polymer is 61.3 wt% VP/38.6 wt% VPA based on titration to pH endpoint of 9.2 (7.15 meq/g Titer). All chemicals were purchased from Sigma-Aldrich unless otherwise noted.

REFERENCE EXAMPLE 2

The anti-erosion efficacy of PVP-PVPA synthesized in Reference Example 1 was compared to commercially available PVP homopolymers (Sigma Aldrich - Cat.# PVP10, PVP4, PVP360) by measuring the pH change due to the dissolution of hydroxyapatite (HAP) powder in citric acid solution. Treatment solutions were prepared by dissolving the PVP homopolymers or PVP-PVPA copolymer in water and adjusting the pH using hydrochloric acid or sodium hydroxide.

In brief, prior to treatment, 100 mg of Ceramic Hydroxyapatite, Type I, 20 μm (Bio-Rad Laboratories, Inc. - Cat. # 158-2000) was incubated for 2 hours at 37°C in (Northeast Laboratory, Waterville, ME - Cat. # T0300) to allow formation of salivary pellicle. Pellicle-coated HAP powder was subjected to one ten-minute treatment mixing end over end with 10 mL of assigned treatment solution. Treated particles were subsequently filtered using a 5 μm pore size, 25 mm diameter, polycarbonate membrane filter (Whatman® Nuclepore™ Track-Etched Membranes - Cat. # 110613) and rinsed with DI water before dispersing into 25 mL of 1 wt% citric acid solution at pH 3.8. The bulk pH value of the citric acid solution was recorded every 30 seconds for 10 minutes. Erosion prevention efficacy was measured from the magnitude of the pH increase from baseline, with lower pH increases indicative of slower dissolution and greater efficacy.

Tables 1 and 2 show the compositions of the various treatment solutions. Table 1 lists solutions with PVP homopolymers, while Table 2 lists solutions with PVP-PVPA copolymers. In some of the solutions, sodium fluoride (NaF) was added to determine if synergistic effects could be found when fluoride ions were in the treatment solution. The tables also show positive controls (100 ppm NaF in water) and negative controls (water).

Table 1: Prototype formulas of PVP homopolymers used in the manual erosion prevention study. Concentrations are listed in % wt/wt.

| **Ingredient** | **A2** | **B2** | **C2** | **D2** | **E2** | **F2** | **G2** | **H2** |
|---|---|---|---|---|---|---|---|---|
| PVP (average mol wt 10,000) | - | - | 2.000 | 2.000 | - | - | - | - |
| PVP (average mol wt 40,000) | - | - | - | - | 2.000 | 2.000 | - | - |
| PVP (average mol wt 360,000) | - | - | - | - | - | - | 2.000 | 2.000 |
| Sodium Fluoride | - | 0.0221 | - | - | - | - | - | - |
| Water, pH adjuster | 100 | QS | QS | QS | QS | QS | QS | QS |
| pH | N/A | 4.2 | 4.2 | 7.0 | 4.2 | 7.0 | 4.2 | 7.0 |
| Note: concentration converted to %w/w assuming bulk product density of 1.000 g/mL.<br>A2 = Water (negative control)<br>B2 = 100 ppm F, pH=4.2 (positive control)<br>C2 = PVP-10K, pH 4.2<br>D2 = PVP-10K, pH 7.0<br>E2 = PVP-40K, pH 4.2<br>F2 = PVP-40K, pH 7.0<br>G2 = PVP-360K, pH 4.2<br>H2 = PVP-360K, pH 7.0 | | | | | | | | |

Table 2: Prototype formulas of PVP-PVPA used in the manual erosion prevention study. Concentrations are listed in % wt/wt.

| **Ingredient** | **A2** | **B2** | **I2** | **J2** | **K2** | **L2** |
|---|---|---|---|---|---|---|
| PVP-PVPA | - | - | 3.263 | 3.263 | 3.263 | 3.263 |
| Sodium Fluoride | - | 0.0221 | - | - | 0.0221 | 0.0221 |
| Water, pH adjuster | 100 | QS | QS | QS | QS | QS |
| pH | N/A | 4.2 | 4.2 | 6.8 | 4.2 | 6.8 |
| Note: concentration converted to %w/w assuming bulk product density of 1.000 g/mL.<br>A2 = Water (negative control)<br>B2 = 100 ppm F, pH 4.2 (positive control)<br>I2 = PVP-PVPA, pH=4.2<br>J2 = PVP-PVPA, pH=6.8<br>K2 = PVP-PVPA + 100 ppm F, pH=4.2<br>L2 = PVP-PVPA +100 ppm F, pH=6.8 | | | | | | |

Table 3 summarizes the erosion prevention efficacy of the treatment solutions from Tables 1 and 2.

Table 3: Results of manual erosion prevention study of prototype formulas of PVP homopolymers and PVP-PVPA copolymers, given as the increase in pH of the bulk solution from baseline (ΔpH).

| **Formulation** | **ΔpH** |
|---|---|
| A2 | 0.29 |
| B2 | 0.09 |
| C2 | 0.26 |
| D2 | 0.29 |
| E2 | 0.28 |
| F2 | 0.28 |
| G2 | 0.30 |
| H2 | 0.28 |
| I2 | 0.26 |
| J2 | 0.23 |
| K2 | 0.09 |
| L2 | 0.05 |

Table 3 shows that the treatment solutions containing only PVP (C2 thru H2) offer insignificant erosion prevention when compared to water alone (ΔpH or 0.29 for water versus 0.26 to 0.30 for PVP treatment solutions). The treatment solutions showing the highest erosion prevention efficacy were those where PVP-PVPA combined with fluoride at pH 4.2 (K2) or at pH of 6.8 (L2).

**EXAMPLE 3**

A second anionic copolymer of vinyl pyrrolidone (anionic PVP-copolymer) was prepared via copolymerization of VP with methacryloxyethyl phosphate acid (MOEP) and hydroxyethyl acrylate via radical polymerization. The resultant copolymer is referred to as (PVP-MOEP).

Both a high and low phosphate PVP-MOEP were prepared, by varying the ratios of the added monomers. Briefly, isopropyl alcohol (371.5 g) was heated to approximately 80° C in a round bottom flask under nitrogen atmosphere. A monomer/initiator solution consisting of IPA (200 g), MOEP, hydroxyethyl acrylate (HEA), VP and VAZO 52 (3 g, Chemours Inc.) was added gradually over 120 minutes. For the high phosphonic acid PVP-MOEP the quantities of the monomers were 120 g MOEP, 25 g HEA, and 55 g VP. For the low low phosphate PVP-MOEP the quantities of the monomers were 68.44 g MOEP, 13 g HEA, and 118.55 g VP. Following addition of the monomer/initiator solution the reaction temperature

was allowed to rise during the polymerization to 83-84°C and maintained at this temperature for an additional 30 minutes before cooling to 77° C. An initiator solution consisting of VAZO 52 (1.5 g) in IPA (10 g) was added and the reaction mass is maintained at 77°C for an additional hour. The polymer solution was cooled, precipitated from hexane, and dried overnight under vacuum. All chemicals were purchased from Sigma-Aldrich unless otherwise noted.

The theoretical composition of the high low phosphate PVP-MOEP was 60 wt% MOEP/12.5wt% HEA/ 27.5wt% NVP, corresponding 4.73 meq/g of titer for titration to a pH endpoint of 9.2. The actual titer required was 5.23 meq/g. The theoretical composition of the low low phosphate PVP-MOEP was 34.2 wt% MOEP/6.5wt% HEA/ 59.3wt% NVP, corresponding 2.70 meq/g of titer for titration to a pH endpoint of 9.2. The actual titer required was 4.63 meq/g.

## EXAMPLE 4

The influence of the high phosphate copolymer (PVP-MOEP) synthesized in Example 3 on erosion prevention was assessed using a constant-composition based auto-titration method. Treatment solutions comprising PVP-MOEP were compared to treatment solutions comprising a commercially available PVP homopolymer. Treatment solutions were prepared by dissolving PVP homopolymers or PVP-MOEP copolymer in water and adjusting the pH to 6.5 using hydrochloric acid or sodium hydroxide.

In brief, prior to treatment Ca-deficient hydroxyapatite disks (HiMed, Old Bethpage NY) were soaked overnight in 10 mL of artificial complete saliva (Northeast Laboratory, Waterville, ME - Cat. # T0300) to form a pellicle layer. Pellicle coated hydroxyapatite was then subjected to one ten-minute treatment, rinsed, and added to a solution of citric acid (1 wt%, pH = 3.2). The pH and calcium activity were measured and maintained at a constant level by auto-titration (Metrohm Titrando). Greater erosion prevention efficacy was correlated with lower volumes of titrant added to maintain constant composition.

Table 4 shows the compositions of the various treatment solutions. Sodium fluoride (NaF) was added to all treatment solution. The tables also show positive controls (0.0221 NaF in water) and negative controls (water).

Table 4. Prototype formulas of PVP-MOEP used in constant composition experiments. Concentrations are listed in % wt/wt. *Plasdone K-29/32 polymer from Ashland, a commercially available water-soluble homopolymer of N-vinyl-2-pyrrolidone with a typical molecular weight of 58,000 daltons.

| Ingredient | A2 | B2 | C4 | D4 | E4 | F4 | G4 | H4 | I4 | J4 |
|---|---|---|---|---|---|---|---|---|---|---|
| PVP-MOEP | - | - | - | - | 0.100 | 0.500 | 1.000 | 1.000 | 1.000 | 1.000 |
| PVP* | - | - | - | 0.275 | - | - | - | - | - | - |
| Sodium Fluoride | - | 0.0221 | 0.0221 | 0.0221 | 0.0221 | 0.221 | 0.0221 | - | 0.0221 | - |
| Water, pH adjuster | 100 | QS | QS | QS | QS | QS | QS | QS | QS | QS |
| pH | N/A | 4.2 | 6.5 | 6.5 | 6.5 | 6.5 | 6.5 | 6.5 | 4.2 | 4.2 |

Table 5 summarizes the results of the constant composition experiments for the treatment solutions from Table 4.

Table 5: Results of constant composition experiments given as the final titrant volume (in mls) after 30 min of auto-titration.

| Formulation | Final Titrant Volume (mean±SE, mls) |
|---|---|
| A2 | 64 ± 2 |
| B2 | 30 ± 1 |
| C4 | 44 ± 5 |
| D4 | 45 ± 2 |
| E4 | 40 ± 1 |
| F4 | 34.4 ± 0.3 |
| G4 | 26 ± 2 |
| H4 | 61 ± 4 |
| I4 | 23 ± 1 |
| J4 | 57 ± 5 |

The table shows that Final Titrate Volume of treatment solutions with increasing concentration of PVP-MOEP with 100 ppm F results in significant erosion prevention. At 1% PVP-MOEP and pH 6.5 the rate of hydroxyapatite dissolution is reduced by 41% relative to treatment with fluoride alone (C4). At 1% PVP-MOEP and pH 4.2 the rate of hydroxyapatite dissolution is reduced by 23% relative to treatment with fluoride alone (B2).

## EXAMPLE 5

In vitro remineralization efficacy of PVP-MOEP prototype formulas was evaluated based on five-day remineralization-demineralization cycling. The PVP-MOEP used in this example was the high phosphate PVP-MOEP synthesized in Example 3.

Briefly, thirty ground and polished human enamel specimens were demineralized to create non-cavitated artificial lesions. Artificial lesions were formed through immersion of specimens in a demineralization solution and the baseline lesion surface ranged 25-45 in units of Vickers hardness number (VHN). Initial surface microhardness was measured for each specimen using a Shimadzu Micro Hardness Tester. The thirty specimens were divided into six groups of six, with the six groups being balanced by the initial surface microhardness of the specimens.

The six treatment groups were subjected to a series of treatments followed by remineralization and demineralization steps. The formulations used were those from Table 4 of Example 4. In brief, A2 was water (negative control), C4 was 100 ppm F (positive control), D4 was 0.275% PVP homopolyer with 100 ppm F, E4 was 0.1% PVP-MOEP with 100 ppm F, F4 was 0.5% PVP-MOEP with 100 ppm F, and G4 was 1.0% PVP-MOEP with 100 ppm F.

The remineralization step used artificial saliva (130 mM KCl, 1.5 mM $CaCl_2$, 0.9 mM $KH_2PO_4$, 25 mM BTP buffer, 2.2 g/L Porcine Mucin, pH =7), while the demineralization step used lactic acid (0.1 M lactic acid, 0.2 wt% Carbopol, 50% saturated with hydroxyapatite, pH = 5.0). The daily regimen, followed for five days, is given in Table 6.

Table 6: Daily regimen for five-day remineralization-demineralization cycling study. *At the start of the study the first treatment is preceded by one hour soak in artificial saliva. ** On day five of the study the final treatment is followed by one hour soak in artificial saliva.

| | |
|---|---|
| 00:02 | Treatment* |
| 01:28 | Artificial Saliva Soak |
| 00:02 | Treatment |
| 01:28 | Artificial Saliva Soak |
| 02:00 | Lactic Acid Challenge |
| 01:30 | Artificial Saliva Soak |
| 00:02 | Treatment |
| 01:28 | Artificial Saliva Soak |
| 00:02 | Treatment |
| 15:58 | Overnight Artificial Saliva Soak** |

After five days, specimens were evaluated for change in surface microhardness (ΔSMH) measured using a Shimadzu Micro Hardness Tester in units of Vickers hardness number (VHN). Higher remineralization efficacy was correlated with a greater increase in surface microhardness.

Table 7: Change in surface microhardness following five-day remin/demin cycling.

| **Treatments** | **Δ SMH (VHN)** |
|---|---|
| A2 | -6 ± 3 |
| C4 | 6 ± 4 |
| D4 | 0.2 ± 4 |
| E4 | 6 ± 3 |
| F4 | 10 ± 4 |
| G4 | 15 ± 5 |

Table 7 shows that following 20 treatments over five days, an increase in the surface microhardness of enamel specimens treated with PVP-MOEP and 100 ppm F was observed. Treatment with 1 wt% PVP-MOEP (G4) resulted in a ΔSMH significantly higher than treatment with fluoride alone (C4).

## EXAMPLE 6

A 5-day cyclic *in vitro* assay was designed to quantify the extent of stain prevention from an instant coffee (10% w/v in water) and black tea (2.0% w/v in H2O) staining solution onto hydroxyapatite (HAP) disks. The completely crystalline HAP disks (12.3mm diameter by 1 mm thick, Himed, Old Bethpage, NY) were initially etched with 5% citric acid for 4 min to make them more amenable to stain formation. Then discs were incubated in artificial complete saliva for hours at 37°C to develop a proteinaceous surface pellicle layer.

During the 5-day cycle, the pristine HAP disks were subjected to two treatments and two stain exposures in the morning and afternoon. Each treatment step was followed by a 5 min exposure to complete artificial saliva (Northeast Laboratory Services, Waterville ME) which was followed by exposure to the staining solution. All exposures to staining and complete saliva solutions were performed at 37°C with agitation. Upon completion of the 5-day cycle all disks were analyzed spectrophotometrically, and the data were reported in terms of L*a*b* color space where L* is the extent of lightness (*i.e.* amount of surface stain), while a* and b* describe the green/red and yellow/blue transitions respectively.

Table 8 shows the compositions of the treatment formulations. Treatment solutions with high phosphate PVP-MOEP were compared to those with PVP homopolymers and those with no polymer.

Table 8: Prototype formulas of PVP-MOEP used in stain prevention experiments. Concentrations are listed in % wt/wt.

| Ingredient | A6 | B6 | C6 |
|---|---|---|---|
| PVP-MOEP | - | - | 2 |
| PVP | - | 2 | - |
| Water, pH adjuster | 100 | Q.S. | Q.S. |
| pH | 7 | 7 | 7 |

Table 9 shows the change in lightness (ΔL*) at the end of the five day stain prevention study. Treatment with the PVP-MOEP copolymer (C6) resulted in significantly reduced loss of lightness relative to PVP, demonstrating its improved stain prevention efficacy.

Table 9: Change in lightness of HAP disks after five-day stain prevention study.

| | A6 | B6 | C6 |
|---|---|---|---|
| ΔL* | -9.78 $\pm$ 4.12 | -8.77 $\pm$ 2.64 | -5.75 $\pm$ 1.44 |

## EXAMPLE 7

Solutions of 1 wt% high phosphate acid PVP-MOEP diluted in commercially available mouthwash formulations were prepared and their stability tested following (i) equilibrium at room temperature, (ii) equilibrium at 40°C, and (iii) freeze-thaw cycling. The commercially available formulations tested are given in Table 10, and represent both alcohol and alcohol-free formulations, and fluoride and non-fluoride containing formulations. All solutions were initially homogenous throughout. Failed stability was identified as the loss of this homogeneity, for example due to the formation of a precipitate. All solutions had passing stability after 31 weeks storage at room temperature and 40°C. Additionally, all solutions had passing stability after three freeze-thaw cycles of 24 hours at -10°C and 24 hours at 25°C.

Table 10: Commercially available mouthwash formulation used to test the stability of PVP-MOEP

| Product Name | Country of Origin | Contains Alcohol | Contains Fluoride |
|---|---|---|---|
| LISTERINE ZERO mouthwash | United States | No | No |
| LISTERINE ZERO mouthwash | Germany | No | Yes |
| LISTERINE COOL MINT mouthwash | United States | Yes | No |

(continued)

| Product Name | Country of Origin | Contains Alcohol | Contains Fluoride |
|---|---|---|---|
| LISTERINE TOTAL CARE ENAMEL GUARD mouthwash | United Kingdom | Yes | Yes |

**Claims**

1. A composition comprising an orally-acceptable carrier and an anionic copolymer derived from the polymerization of N-vinyl pyrrolidone (VP) with an anionic monomer compound containing phosphorus, wherein said anionic copolymer is described by Formula I below:

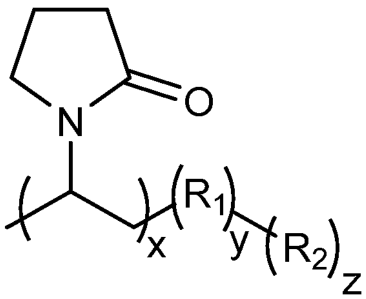
(I)

wherein x, y, and z are integers and the ratio of "x" to "y+z" is from 1:99 to 99:1; $R_1$ is derived from methacryloxyethyl phosphate; and
$R_2$ is a repeat unit selected from hydroxyethyl acrylate (HEA), 2-hydroxypropyl acrylate, 4-hydroxybutyl acrylate, 2-Hydroxyethyl methacrylate, Hydroxypolyethoxy allyl ether, 3-Phenoxy 2 hydroxy propyl methacrylate, glycerol monomethyacrylate and N-(2-Hydroxypropyl)methacrylamide;

wherein the composition further comprises fluoride.

2. The composition of claim 1 wherein z is non-zero and R2 is derived from HEA.

3. The composition of claim 1 comprising from 0.01 to 2% of said anionic copolymer.

4. The composition of claim 1 wherein said composition is in a form selected form the group consisting of a mouthwash, mouth rinse, mouth spray, toothpaste, tooth gel, sub-gingival gel, mousse, foam, denture care product, dentifrice, lozenge, concentrate and chewable tablet.

5. The composition of claim 4 wherein said composition is a mouthwash comprising water, and at least one surfactant selected from the group consisting of anionic, cationic, non-ionic, zwitterionic surfactants and combinations of two or more thereof.

6. The composition of claim 5 further comprising at least one essential oil selected from the group consisting of menthol, thymol, eucalyptol, methyl salicylate, and combinations of two or more thereof.

7. The composition of claim 1 wherein said copolymer has a weight average molecular weight of from 10,000 to 1,000,000.

8. The composition of claim 7 wherein said copolymer has a weight average molecular weight of from 10,000 to 500,000.

9. The composition of any preceding claim for use in a method of inhibiting demineralization of a tooth by contacting a tooth surface with said composition.

**Patentansprüche**

1. Zusammensetzung, umfassend einen oral akzeptablen Träger und ein anionisches Copolymer, das aus der Polymerisation von N-Vinylpyrrolidon (VP) mit einer phosphorhaltigen anionischen Monomerverbindung stammt, wobei das anionische Copolymer durch die nachstehende Formel I beschrieben ist:

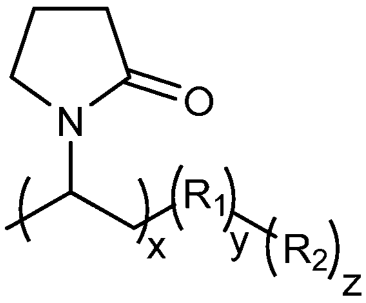

(I)

   wobei x, y und z ganze Zahlen sind und das Verhältnis von "x" zu "y+z" 1:99 bis 99:1 beträgt;
   $R_1$ von Methacryloxyethylphosphat abgeleitet ist; und
   $R_2$ für eine Wiederholungseinheit steht, die aus Hydroxyethylacrylat (HEA), 2-Hydroxypropylacrylat, 4-Hydroxybutylacrylat, 2-Hydroxyethylmethacrylat, Hydroxypolyethoxyallylether, 3-Phenoxy-2-hydroxypropylmethacrylat, Glycerinmonomethylacrylat und N-(2-Hydroxypropyl)methacrylamid ausgewählt ist;
   wobei die Zusammensetzung ferner Fluorid umfasst.

2. Zusammensetzung nach Anspruch 1, wobei z ungleich Null ist und R2 von HEA abgeleitet ist.

3. Zusammensetzung nach Anspruch 1, umfassend 0,01 bis 2 % des anionischen Copolymers.

4. Zusammensetzung nach Anspruch 1, wobei die Zusammensetzung in einer Form vorliegt, die aus der Gruppe bestehend aus einem Mundwasser, einer Mundspülung, einem Mundspray, einer Zahnpasta, einem Zahngel, einem Subgingivalgel, einer Mousse, einem Schaum, einem Zahnpflegemittel, einem Zahnputzmittel, einer Lutschtablette, einem Konzentrat und einer Kautablette ausgewählt ist.

5. Zusammensetzung nach Anspruch 4, wobei es sich bei der Zusammensetzung um ein Mundwasser handelt, das Wasser und mindestens ein Tensid, das aus der Gruppe bestehend aus anionischen, kationischen, nichtionischen, zwitterionischen Tensiden und Kombinationen von zwei oder mehr davon ausgewählt ist, umfasst.

6. Zusammensetzung nach Anspruch 5, ferner umfassend mindestens ein ätherisches Öl, das aus der Gruppe bestehend aus Menthol, Thymol, Eukalyptol, Methylsalicylat und Kombinationen von zwei oder mehr davon ausgewählt ist.

7. Zusammensetzung nach Anspruch 1, wobei das Copolymer ein gewichtsmittleres Molekulargewicht von 10.000 bis 1.000.000 aufweist.

8. Zusammensetzung nach Anspruch 7, wobei das Copolymer ein gewichtsmittleres Molekulargewicht von 10.000 bis 500.000 aufweist.

9. Zusammensetzung nach einem vorhergehenden Anspruch zur Verwendung bei einem Verfahren zur Hemmung der Demineralisierung eines Zahns durch Inkontaktbringen einer Zahnoberfläche mit der Zusammensetzung.

**Revendications**

1. Composition comprenant un support oralement acceptable et un copolymère anionique dérivé de la polymérisation de N-vinylpyrrolidone (VP) avec un composé monomérique anionique contenant du phosphore, dans laquelle ledit copolymère anionique est décrit par la formule I suivante :

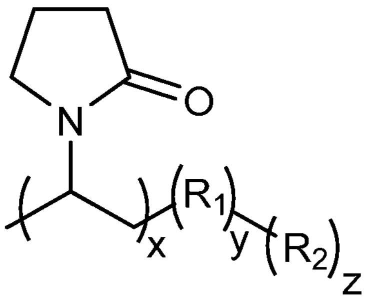

(I)

dans laquelle x, y et z sont des entiers et le rapport de « x » sur « y+z » est de 1:99 à 99:1 ;
$R_1$ est dérivé du phosphate de méthacryloxyéthyle ; et
$R_2$ est un motif répétitif choisi parmi l'acrylate d'hydroxyéthyle (HEA), l'acrylate de 2-hydroxypropyle, l'acrylate de 4-hydroxybutyle, le méthacrylate de 2-hydroxyéthyle, l'hydroxypolyéthoxyallyléther, le méthacrylate de 3-phénoxy-2-hydroxypropyle, le monométhacrylate de glycérol et le N-(2-hydroxypropyl)méthacrylamide ;
dans laquelle la composition comprend en outre du fluorure.

2. Composition selon la revendication 1, dans laquelle z est non nul et R2 est dérivé de HEA.

3. Composition selon la revendication 1, comprenant de 0,01 à 2 % dudit copolymère anionique.

4. Composition selon la revendication 1, dans laquelle ladite composition est sous une forme choisie dans le groupe constitué d'un bain de bouche, un rinçage buccal, un spray buccal, une pâte dentifrice, un gel dentaire, un gel sous-gingival, une mousse, une mousse, un produit de soin de dentier, un dentifrice, une pastille, un concentré et un comprimé à mâcher.

5. Composition selon la revendication 4, dans laquelle ladite composition est un bain de bouche comprenant de l'eau, et au moins un tensioactif choisi dans le groupe constitué des tensioactifs anioniques, cationiques, non ioniques, zwitterioniques et des combinaisons de deux ou plusieurs de ceux-ci.

6. Composition selon la revendication 5, comprenant en outre au moins une huile essentielle choisie dans le groupe constitué du menthol, du thymol, de l'eucalyptol, du salicylate de méthyle et des combinaisons de deux ou plusieurs de ceux-ci.

7. Composition selon la revendication 1, dans laquelle ledit copolymère a un poids moléculaire moyen en poids de 10 000 à 1 000 000.

8. Composition selon la revendication 7, dans laquelle ledit copolymère a un poids moléculaire moyen en poids de 10 000 à 500 000.

9. Composition selon l'une quelconque des revendications précédentes, destinée à être utilisée dans un procédé d'inhibition de la déminéralisation d'une dent en mettant en contact une surface de dent avec ladite composition.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005027862 A1
- US 2008305457 A1
- WO 2016173871 A1
- JP 2992415 B
- JP H06157270 A
- EP 0897709 A1
- US 20060013778 A, Hodosh
- US 6416745 B, Markowitz
- US 7417020 B, Fevola
- US 7087650 B, Lennon
- US 7084104 B, Martin
- US 5190747 A, Sekiguchi
- US 4051234 A, Gieske
- US 5328682 A, Pullen
- US 4994262 A
- US 5626838 A
- US 3678154 A, Widder
- US 5338537 A, White, Jr.
- US 4847070 A, Pyrz
- US 4661341 A, Benedict
- US 4775525 A, Pera
- GB 741315 A
- WO 9912517 A
- US 5538714 A, Pink
- US 5670138 A, Venema
- US 4138477 A, Gaffar
- US 4183914 A, Gaffar
- US 5015467 A, Smitherman
- US 5849271 A
- US 5622689 A, Lukacovic
- US 6682722 B, Majeti
- US 6121315 A, Nair